**Europäisches Patentamt**

**European Patent Office**

**Office européen des brevets**

(19)

(11) Veröffentlichungsnummer : **0 127 052 B2**

(12) **NEUE EUROPÄISCHE PATENTSCHRIFT**

(45) Veröffentlichungstag der neuen Patentschrift :
09.11.94 Patentblatt 94/45

(51) Int. Cl.⁵ : **H01B 7/28**

(21) Anmeldenummer : **84105463.8**

(22) Anmeldetag : **14.05.84**

(54) **Elektrische Isolierungen.**

(30) Priorität : **25.05.83 DE 3318988**

(43) Veröffentlichungstag der Anmeldung :
**05.12.84 Patentblatt 84/49**

(45) Bekanntmachung des Hinweises auf die
Patenterteilung :
**04.10.89 Patentblatt 89/40**

(45) Bekanntmachung des Hinweises auf die
Entscheidung über den Einspruch :
**09.11.94 Patentblatt 94/45**

(84) Benannte Vertragsstaaten :
**AT BE CH DE FR GB IT LI NL SE**

(56) Entgegenhaltungen :
**BE-A- 786 958**
**DE-A- 2 737 430**
**DE-A- 2 805 875**
**GB-A- 1 303 432**
**US-A- 4 198 310**
**Firmenschrift Wacker Chemie GmbH, Wacker
HDK, Sept 1975**

(56) Entgegenhaltungen :
**Firmenschrift Union Carbide, Kabel Items No.
150, Seiten 3-21**
**IEEE TRANSACTIONS ON ELECTRICAL IN-
SULATION, Vol. EI-15, No. 3, June 1980, Seiten
251-258**
**1980 Annual Report, Conference on Electrical
Insulation and Dielectric Phenomena, National Academy Press, Washington D.C. 1980,
Seiten 203-210**
**1969 Annual REport Conference on Electrical
Insulation and Dielectric Phenomena, National Academy of SCience, Washington D.C.,
1970, Seiten 128-136**

(73) Patentinhaber : **SIEMENS
AKTIENGESELLSCHAFT
Wittelsbacherplatz 2
D-80333 München (DE)**

(72) Erfinder : **Henkel, Hans-Joachim, Dr.
Philip-Reis-Strasse 34
D-8520 Erlangen (DE)**
Erfinder : **Müller, Norbert, Dr.
von Hauck-Strasse 15 b
D-8522 Herzogenaurach (DE)**

EP 0 127 052 B2

## Beschreibung

Die Erfindung betrifft elektrische Isolierungen auf Polyolefinbasis, insbesondere bei Kabeln und Leitungen, für Mittel- und Hochspannung ab ca. 10 kV mit einem Zusatz zum Retardieren von Wasserbäumchen.

In elektrisch beanspruchten Polyolefinisolierungen können Vorgänge ablaufen die als "electrochemical treeing" (ECT) oder "water treeing" bezeichnet werden. Diese Vorgänge, die insbesondere im Hinblick auf die Betriebssicherheit kunststoffisolierter Mittel- und Hochspannungskabel von Bedeutung sind, führen zur Entstehung von bäumchenartigen Gebilden, den sogenannten ECT-Strukturen.

Das optische Erscheinungsbild von ECT-Strukturen, die nach geeigneter Anfärbung besonders kontrastreich und detailliert sichtbar sind, ist sehr vielfältig. Grundsätzlich unterscheidet man zwischen zwei Formen:
- "vented trees", die von der Oberfläche der Isolierung ausgehen und sich in die Isolierung hinein erstrecken, und
- "bow-tie trees", die im Inneren der Isolierung entstehen.

Der Mechanismus der ECT-Bildung ist bislang nicht geklärt. Allgemein wird aber angenommen, daß für die Bildung der ECT-Strukturen ein elektrisches Feld und die Anwesenheit einer polaren Flüssigkeit, insbesondere von Wasser, erforderlich ist; die ECT-Strukturen werden deshalb auch als Wasserbäumchen bezeichnet. Die Initiierungsorte der Wasserbäumchen scheinen immer Störstellen zu sein, wie Verunreinigungen, aggregierte Beimischungen, Hohlräume, Spalte, Risse oder Grenzflächen, von denen jedoch jeweils nur ein Teil zur Bildung von Wasserbäumchen führt. Von den Störstellen aus, die bei im großtechnischen Maßstab hergestellten Isolierungen nicht vollständig vermieden werden können, erstrecken sich die bäumchenartigen Strukturen in Richtung des elektrischen Feldes.

Da ECT-Strukturen lokale Veränderungen des Isoliermaterials darstellen, können sie eine Schädigung der Isolierung bewirken, insbesondere im Hinblick auf die elektrische Durchschlagsfestigkeit. Es sind deshalb bereits zahlreiche Versuche unternommen worden, um das Wachstum von Wasserbäumchen zu verhindern oder zumindest zu verzögern.

Eine der Maßnahmen zur Verhinderung der Bildung von Wasserbäumchen besteht darin, die Isolierschicht mit einer metallischen Umhüllung bzw. Ummantelung, beispielsweise aus Blei oder Aluminium, zu versehen Kabel mit einer derartigen Wasserabschirmschicht sind aber nicht nur kostspieliger, sondern auch schwerer und deshalb auch schwieriger handzuhaben als Kabel ohne eine Metallhülle.

Aus diesem Grunde ist deshalb bereits versucht worden, die Bildung von ECT-Strukturen durch die Zugabe von Additiven zur Isolierschicht (oder angrenzenden Schichten) zu unterbinden. Aus der Vielzahl der dabei eingesetzten Verbindungen seien beispielhaft genannt: Bleistearat (DE-OS-2 425 760 bzw. GB-PS-1 473 867), Natriumchlorid und -sulfat oder andere starke Elektrolyte (DE-AS-2 537 283 bzw. US-PS-4 042 776), stabile Hydrate bildende Salze, wie Calcium- und Magnesiumchlorid, und basische Anhydride (DE-OS-2 817 804 bzw GB-PS-1 584 501), Silicagel und Phosphorpentoxid (DE-OS-2 754 336), Organosilane (US-Patentschriften 4 144 202, 4 212 756 und 4 263 158 sowie DE-OS-2 737 430 und DE-OS-2 805 875), Bleioxide und basische Bleiverbindungen (DE-OS-2 523 844 und DE-OS-2 806 752), organische Isocyanate (US-PS-4 282 333), auf das Polymermaterial aufgepfropfte Silanverbindungen (DE-OS-2 935 224) und Metallkomplexe von Diketonen, Salicylsäure oder Schiffschen Basen (EP-A1-27 300).

Andererseits und konträr zu den Vorschlägen, salzartige Verbindungen bzw. Elektrolyte zuzusetzen, ist aber auch schon vorgeschlagen worden, den Gehalt der Isolierung an darin fein verteilten wasserlöslichen und/oder hygroskopischen Salzen unter einem Wert von $10^{-1}$ ppm und vorzugsweise unter $10^{-4}$ ppm zu halten (DE-OS-2 911 756).

Offensichtlich haben aber all diese Maßnahmen, die sich darüber hinaus zum Teil auch widersprechen, noch nicht den erwünschten Erfolg gebracht. Denn so findet sich in einem Bericht über die Cigré, d. h. die Internationale Hochspannungskonferenz, von 1980 der Hinweis (siehe: "Kunststoffe" 71, 1981, Seite 448 ff), daß empfohlen wurde, bei (Hochspannungs-)Kabeln einen wasserdichten Metallschirm aufzubringen, um das Eindringen von Feuchtigkeit zu verhindern.

Aufgabe der Erfindung ist es, bei elektrischen Isolierungen der eingangs genannten Art, d. h. auf der Basis vernetzter und unvernetzter Polyolefine, das Wachstum von Wasserbäumchen durch einen geeigneten Zusatz wirksam und dauerhaft zu unterdrücken bzw. deren Bildung so weit wie möglich zu verhindern, so daß auf aufwendige Maßnahmen, wie Metallmäntel, verzichtet werden kann.

Dies wird erfindungsgemäß dadurch erreicht, daß der Zusatz ausschließlich aus einem in homogener Verteilung mit einem Anteil von 0,1 bis 4 Gew.-%, bezogen auf das Gesamtgewicht, vorliegenden hydrophilen, für Schwermetallionen adsorptionsaktiven oder Schwermetallionen im Ionenaustausch bindenden Stoff mit einer Partikelgröße bis zu 50 μm bzw. einer Agglomeratgröße bis zu 100 μm besteht.

Gegenstand der Erfindung sind ferner Kabel und Leitungen für Mittel- und Hochspannung ab ca. 10 kV mit einer einen Zusatz zum Retardieren von Wasserbäumchen enthaltenden elektrischen Isolierung auf Polyolefinbasis. Diese Kabel und Leitungen sind dadurch gekennzeichnet, daß der Zusatz ausschließlich aus einem in homogener Verteilung mit einem Anteil von 0,1 bis 4 Gew.-%, bezogen auf das Gesamtgewicht der Isolierung, vorliegenden hydrophilen, für Schwermetallionen adsorptionsaktiven oder Schwermetallionen im Ionenaustausch bindenden Stoff mit einer Partikelgröße bis zu 50 μm bzw. einer Agglomeratgröße bis zu 100 μm besteht.

Unter einer "homogenen Verteilung" wird im Rahmen der vorliegenden Patentanmeldung die Tatsache verstanden, daß keine besonderen Maßnahmen zur Beeinflussung der sich beim Verarbeitungsprozeß ergebenden Verteilung nötig sind bzw. ergriffen werden.

Vorzugsweise ist in den erfindungsgemäßen elektrischen Isolierungen als Zusatz pyrogene oder gefällte Kieselsäure enthalten. Als Zusatz kann vorteilhaft aber auch Aluminiumoxid und -oxidhydrat mit großer aktiver Oberfläche oder Aluminiumsilicat Verwendung finden. Bei dem Zusatz handelt es sich dabei vorzugsweise um ein synthetisches Produkt. Es kann aber auch ein natürlich vorkommendes Mineral eingesetzt werden, und zwar dann, wenn es hohen Anforderungen an Reinheit und Gleichmäßigkeit der Partikelgröße entspricht; das Mineral soll dabei möglichst wenig Gangart und möglichst wenig Spurenverunreinigungen enthalten. Aber auch allgemein soll der Zusatz möglichst rein sein.

Die in den erfindungsgemäßen elektrischen Isolierungen enthaltenen Kieselsäuren sind vorzugsweise synthetische Produkte, die in hochdisperser, d. h. feinteiliger Form vorliegen; diese Kieselsäuren (chemische Zusammensetzung: $SiO_2 \cdot x\ H_2O$) zeichnen sich auch dadurch aus, daß sie nicht kristallin sind, sondern röntgenamorph. Synthetische Kieselsäuren werden im allgemeinen nach pyrogenen Verfahren oder nach nassen Verfahren hergestellt. Pyrogene Kieselsäure entsteht beispielsweise bei der Hydrolyse von Siliciumtetrachlorid in einer Knallgasflamme (Temperatur: ca. 1000° C). Gefällte Kieselsäure (oder Fällungskieselsäure) wird aus Natriumsilicatlösungen durch Zugabe von Säuren, wie Salz- oder Schwefelsäure, hergestellt; das Fällungsprodukt wird dabei gewaschen, getrocknet und auf 300 bis 800° C, vorzugsweise 400 bis 700° C, erhitzt und anschließend gemahlen.

Die in den elektrischen Isolierungen enthaltenen Aluminiumoxide und -oxidhydrate können in Form natürlich vorkommender Mineralien eingesetzt werden, wie AlO(OH) bzw. $Al_2O_3 \cdot H_2O$. Die Oxide und Oxidhydrate können aber auch - nach bekannten Verfahren - synthetisiert werden. Dazu werden beispielsweise aus Aluminiumsulfatlösungen mittels Ammoniak Oxidhydrate ausgefällt und getrocknet und anschließend auf Temperaturen von 400 bis 750° C, vorzugsweise 500 bis 600° C, erhitzt und dann gemahlen.

Die Aluminiumsilicate können ebenfalls in Form von (gereinigten) natürlichen Mineralprodukten eingesetzt werden, wie Kaolinit $Al_4(OH)_8[Si_4O_{10}]$ bzw. $Al_2O_3 \cdot 2\ SiO_2 \cdot 2\ H_2O$, Montmorillonit $Al_2(OH)_2[Si_4O_{10}]$ bzw $Al_2O_3 \cdot 4\ SiO_2 \cdot H_2O$ und Bentonit, einem Tonmineral mit Montmorillonit als Hauptbestandteil. Die Aluminiumsilicate können aber ebenfalls nach bekannten Verfahren aus Lösungen gefällt werden; nach dem Trocknen werden sie dann auf 350 bis 950° C, vorzugsweise 450 bis 700° C, erhitzt und anschließend gemahlen.

Mittels des erfindungsgemäßen Zusatzes kann einerseits die ECT-Bildung in elektrischen Isoliermaterialien unterdrückt werden, andererseits wird durch diese Verbindung das Angebot an wirksamen Zusätzen erweitert.

Dieses Angebot besteht bislang in Barbitursäure bzw. 2-Thiobarbitursäure und Derivaten davon (deutsche Offenlegungsschrift 3 202 828) sowie in wasserlöslichen Alkali- oder Erdalkaliphosphaten und hydrolysierbaren Phosphorsäureestern (deutsche Offenlegungsschrift 3 202 896).

Aus der bereits genannten DE-OS-2 754 336 ist ein Hochspannungskabel mit einer Leiterisolierung aus Kunststoff, insbesondere aus einem Polyolefin, bekannt, bei welchem in der Kunststoffisolierung von innen nach außen ein Diffusionsgefälle besteht. Durch diese Maßnahme soll erreicht werden, daß in der extrudierten Leiterisolierung die Bildung und das Wachstum von Wasserbäumchen möglichst zuverlässig unterbunden wird. Zur Erzeugung eines Diffusionsgefälles in der Leiterisolierung sind dabei eine Reihe von Möglichkeiten angegeben, wie Umgeben der Leiterisolierung mit einer Schicht aus wasserabsorbierendem Material oder aus einem Material mit einem hohen Verlustfaktor, Einbringen eines wasserabsorbierenden Stoffes in die Isolierung mit von innen nach außen zunehmender Konzentration (als derartige Stoffe sind Silicagel und Phosphorpentoxid genannt), Beheizen des Leiters oder des die Leiterisolierung umgebenden äußeren Schirmes und Führen eines trockenen Gasstromes zwischen der Leiterisolierung und dem Außenmantel; dabei sollen zweckmäßigerweise mehrere dieser Maßnahmen gleichzeitig angewendet werden. Bezüglich der Konzentration und der Partikelgröße von Silicagel und Phosphorpentoxid ist nichts ausgesagt; im übrigen bringt die Verwendung derartiger wasserabsorbierender Stoffe auch keine dauerhafte Lösung des Problems.

Sämtliche der genannten Maßnahmen sind aber sehr aufwendig; dies gilt auch für die Erzeugung eines Konzentrationsgefälles in der Isolierschicht. Es war deshalb mehr als überraschend und konnte nicht vorhergesehen werden, daß es - auch zur Erzielung einer hohen Wirksamkeit - beispielsweise ausreichend ist, zur Unterbindung der ECT-Bildung als Zusatz pyrogene oder gefällte Kieselsäure zu verwenden und diesen Zusatz im Isoliermaterial homogen zu verteilen. Dies gilt auch in bezug auf die - ebenfalls bereits genannte-DE-OS-2 805 875. Daraus ist es bekannt, extrudierten, leitfähigen Ruß enthaltenden feldbegrenzenden Schichten von Hochspannungskabeln, insbesondere den äußeren Leitschichten, zur Verhinderung der ECT-Bildung Alkoxysilane und adsorptionsaktive Füllstoffe in Form von oberflächenaktivem Ruß, feindisperser gefällter Kieselsäure und/oder synthetischer Kieselsäure zuzusetzen. Der wesentliche Zusatz ist dabei aber das Alkoxysilan, das von dem in das Kabel eindringende Wasser hydrolysiert wird, d. h. zur chemischen Bindung des eindringenden Wassers dient. Das Ziel der genannten Maßnahmen ist nämlich eine Senkung des Feuchtigkeitsgehaltes in der Isolierung. Im Gegensatz dazu wird bei der vorliegenden Patentanmeldung von der Vorstellung ausgegangen, daß in der Isolierung vorhandene Metallionen den ECT-Prozeß fördern, wobei der schädliche Gehalt an Metallionen in der Isolierung gleichmäßig verteilt ist. Diese Metallionen werden durch den erfindungsgemäßen Zusatz gebunden, in der Isolierung vorhandene Feuchtigkeit vermag dann keine ECT-Bildung mehr zu bewirken.

Außer in Kabeln und Leitungen können die erfindungsgemäßen elektrischen Isolierungen auch in Muffen und in Garnituren Verwendung finden. Als Grundlage dienen bei diesen Isoliermaterialien Polyolefine, und zwar vernetzte oder unvernetzte Materialien. Insbesondere finden in den erfindungsgemäßen Isolierungen Polyethylen (PE) und vernetztes Polyethylen (VPE) Verwendung. Daneben können aber auch Ethylen-Copolymere, wie Ethylen-Propylen-Copolymere (EPR), Ethylen-Vinylacetat-Copolymere (EVA) und Ethylen-Alkylacrylat-Copolymere (beispielsweise Ethylen-Ethylacrylat- und -Butylacrylat-Copolymere), bzw. Ethylen-Propylen-Dien-Terpolymere und Gemische (Blends) dieser Ethylen-Copolymere und -Terpolymere mit Polyolefinen, insbesondere Polyethylen und Polypropylen, eingesetzt werden. Die genannten Polymeren bzw. Polymergemische können, wie bereits erwähnt, sowohl vernetzt als auch unvernetzt zum Einsatz kommen. Die Vernetzung erfolgt dabei vorzugsweise peroxidisch oder durch energiereiche Strahlen. Die Isoliermaterialien können gegebenenfalls auch mit Oxidationsstabilisatoren versehen sein.

Der Anteil des Zusatzes liegt, wie bereits ausgeführt, zwischen 0,1 und 4 Gew.-%, bezogen auf das Gesamtgewicht der elektrischen Isolierung. Vorzugsweise liegt der Anteil des Zusatzes bei 0,5 bis 2 Gew.-%. Die Konzentration des Zusatzes ist nach oben dadurch begrenzt, daß der dielektrische Verlustfaktor nicht merklich ansteigen soll. Im übrigen müssen die Spezifikationen nach der IEC-Publikation 502 (Aufgabe 1978) erfüllt sein.

In den erfindungsgemäßen elektrischen Isolierungen beträgt die Partikelgröße des Zusatzes bis zu 50 µm bzw. die Agglomeratgröße bis zu 100 µm. Unter Agglomeraten werden dabei lokere Zusammenlagerungen von Partikeln verstanden, die - beispielsweise durch Scherkräfte - in mehr oder weniger großem Ausmaß zerteilt werden können. Vorteilhaft beträgt die Partikelgröße bis zu 20 µm.

Der Zusatz kann auch zusammen mit sogenannten Phlegmatisierungsmitteln in die Polyolefine eingearbeitet werden, wobei eine Paste aus dem Zusatz und einem Phlegmatisierungsmittel bevorzugt wird. Als Phlegmatisierungsmittel eignen sich insbesondere Pareffine, Paraffinalkohole und Ester, wobei Kohlenwasserstoffe, d. h. unpolare Verbindungen, wegen der Erhaltung der elektrischen Eigenschaften bevorzugt werden.

Anhand von Ausführungsbeispielen soll die Erfindung noch näher erläutert werden.

Zum Nachweis der Verminderung der Bildung von Wasserbäumchen wurden aus hochreinem, unstabilisiertem thermoplastischem Polyethylen niederer Dichte (LDPE) 3 mm dicke Platten - mit und ohne Zusatz - hergestellt. Zur Herstellung von erfindungsgemäßen Isolierungen wurden dabei in einen Teil des Plattenmaterials vor dem Verpressen pyrogene Kieselsäuren mit unterschiedlich aktiver Oberfläche, die unter dem Namen Aerosil ® im Handel erhältlich sind, in Konzentrationen von 0,5 und 1 Gew.-% homogen eingemischt. Die plattenförmigen Prüflinge wurden dann mit 10 kV/50 Hz elektrisch belastet, wobei sich beide Oberflächen in direktem Kontakt mit einer auf 70° C erwärmten 3 %-igen Natriumchloridlösung befanden. Die Belastungsdauer betrug 130 Stunden.

Die Versuchsergebnisse zeigen, daß die pyrogene Kieselsäure enthaltenden Plattenprüflinge - unter gleichen Versuchsbedingungen - im Vergleich zu den Prüflingen ohne Kieselsäurezusatz weniger bzw. gar keine ECT-Strukturen enthalten. Es ergibt sich ferner auch ein beachtlicher Unterschied in der Größe der ECT-Strukturen. Während nämlich die Längenausdehnung der ECT-Strukturen in Richtung des elektrischen Feldes in den Prüflingen ohne Kieselsäurezusatz bis zu 1500 µm beträgt, weisen die kieselsäurehaltigen Prüflinge ECT-Strukturen unterhalb 500 µm auf.

Im einzelnen ergibt sich beispielsweise folgendes:

| Zusatz | | Länge der ECT-Strukturen | relative Anzahl der ECT-Strukturen (bezogen auf Vergleichsversuch) |
|---|---|---|---|
| - (Vergleichsversuch) | | $\leqslant$ 1500 µm | - |
| Aerosil | 130 | $\leqslant$ 500 µm | geringer |
| " | 200 | $\leqslant$ 300 µm | viel geringer |
| " | 300 | - | keine |
| " | 380 | - | keine |

Die vorstehend aufgeführten pyrogene Kieselsäuren weisen - in der angegebenen Reihenfolge - eine zunehmende spezifische Oberfläche auf, gemessen nach der BET-Methode (Aerosil 130 : 130 ± 25 m²/g. Aerosil 380 : 380 ± 30 m²/g). Daraus ergibt sich, daß bei den erfindungsgemäßen elektrischen Isolierungen der Zusatz vorzugsweise eine hohe spezifische Oberfläche besitzt.

Entsprechende Ergebnisse wie bei pyrogener Kieselsäure werden auch beim Zusatz von gefällter Kieselsäure (hydrophil, BET-Oberfläche: 170 m²/g) erzielt. Während nämlich Plattenprüflinge ohne Zusatz, wie bereits ausgeführt, zahlreiche ECT-Strukturen mit einer Länge bis zu 1500 µm enthalten, weisen Prüflinge mit einem Zusatz von gefällter Kieselsäure (Anteil: 0,5 bis 1 Gew.-%) sehr wenige ECT-Strukturen mit einer Länge $\leqq$ 100 µm auf.

Bei den erfindungsgemäßen elektrischen Isolierungen ist wesentlich, daß der Zusatz hydrophil ist, wie dies beispielsweise bei den synthetischen Kieselsäuren der Fall ist. Diese Kieselsäuren weisen nämlich an der Oberfläche SiOH-Gruppen auf. Werden diese Gruppen-beispielsweise durch Umsetzung mit Alkylchlorsilanensubstituiert, so entstehen partiell oder vollständig hydrophobierte Produkte. Ein derartiges Produkt ist beispielsweise das im Handel erhältliche Aerosil R 972. Wird eine derartige Kieselsäure als Zusatz zu elektrischen Isolierungen verwendet, so ergeben sich - bei den vorstehend angegebenen Bedingungengleich viele ECT-Strukturen wie bei Prüflingen ohne Zusatz, wobei aber die Länge der ECT-Strukturen sogar großer ist: Sie beträgt bis zu 2400 µm.

Entsprechendes gilt bei hydrophoben gefällten Kieselsäuren. Prüflinge mit derartigen Kieselsäuren (BET-Oberfläche: 100 m²/g) zeigen dabei sogar eine gegenüber dem Vergleichsversuch erhöhte Anzahl von ECT-Strukturen, noch dazu mit einer größeren Länge ($\leqq$ 1900 µm).

Entsprechende Ergebnisse wie bei Kieselsäuren ergeben sich auch beim Zusatz von Aluminiumoxiden bzw. -oxidhydraten und Aluminiumsilicaten (Konzentration: 0,1 bis 1 Gew.-%). So weisen beispielsweise Prüflinge mit einem Zusatz von Kaolinit oder Metakaolinit (BET-Oberfläche: 70 m²/g) wenige ECT-Strukturen mit einer Länge $\leqq$ 500 µm auf und Prüflinge mit einem Zusatz von synthetischem $\gamma$-Al$_2$O$_3$ (BET-Oberfläche: 220 m²/g) wenige ECT-Strukturen mit einer Länge $\leqq$ 300 µm.

Die an Plattenprüflingen erhaltenen Ergebnisse werden durch Untersuchungen an Kabeln bestätigt. So ergab beispielsweise die Überprüfung eines Versuchskabels mit einem Zusatz von 2 Gew.-% Aerosil 300 zur Isolierung-nach einer Belastungsdauer von 2600 h - nur vereinzelt ECT-Strukturen mit einer Länge bis zu 200 µm, während ein Versuchskabel ohne Zusatz viele ECT-Strukturen mit einer Länge bis zu 1000 µm aufwies.

Es zeigt sich somit, daß die ECT-Bildung durch den erfindungsgemäßen Zusatz beträchtlich reduziert werden kann, und zwar bereits bei geringen Konzentrationen. Dies hat den weiteren Vorteil, daß die elektrischen Eigenschaften der Isolierungen nicht oder nur unwesentlich beeinflußt werden.

**Patentansprüche**

1. Elektrische Isolierungen auf Polyolefinbasis, insbesondere bei Kabeln und Leitungen, für Mittel- und Hochspannung ab ca. 10 kV mit einem Zusatz zum Retardieren von wasserbäumchen, **dadurch gekennzeichnet,** daß der Zusatz ausschließlich aus einem in homogener Verteilung mit einem Anteil von 0,1 bis 4 Gew.-%, bezogen auf das Gesamtgewicht, vorliegenden hydrophilen, für Schwermetallionen adsorptionsaktiven oder Schwermetallionen im Ionenaustausch bindenden Stoff mit einer Partikelgröße bis zu 50 µm bzw. einer Agglomeratgröße bis zu 100 µm besteht.

2. Elektrische Isolierungen nach Anspruch 1, **dadurch gekennzeichnet,** daß der Zusatz pyrogene oder gefällte Kieselsäure ist.

3. Elektrische Isolierungen nach Anspruch 1, **dadurch gekennzeichnet,** daß der Zusatz Aluminiumoxid, Aluminiumoxidhydrat mit großer aktiver Oberfläche oder Aluminiumsilicat ist.

**4.** Elektrische Isolierungen nach einem der Ansprüche 1 bis 3, **dadurch gekennzeichnet,** daß der Zusatz ein synthetisches Produkt ist.

**5.** Elektrische Isolierungen nach einem oder mehreren der Ansprüche 1 bis 4, **dadurch gekennzeichnet,** daß der Anteil des Zusatzes 0,5 bis 2 Gew.-% beträgt.

**6.** Elektrische Isolierungen nach einem oder mehreren der Ansprüche 1 bis 5, **dadurch gekennzeichnet,** daß die Partikelgröße des Zusatzes bis zu 20 µm beträgt.

**7.** Verfahren zur Herstellung von elektrischen Isolierungen nach einem oder mehreren der Ansprüche 1 bis 6, **dadurch gekennzeichnet,** daß der Zusatz zusammen mit Phlegmatisierungsmitteln, vorzugsweise in Form einer Paste, in die Polyolefine eingearbeitet wird.

**8.** Kabel oder Leitung für Mittel- und Hochspannung ab ca. 10 kV mit einer einen Zusatz zum Retardieren von Wasserbäumchen enthaltenden elektrischen Isolierung auf Polyolefinbasis, **dadurch gekennzeichnet,** daß der Zusatz ausschließlich aus einem in homogener Verteilung mit einem Anteil von 0,1 bis 4 Gew.-%, bezogen auf das Gesamtgewicht der Isolierung, vorliegenden hydrophilen, für Schwermetallionen adsorptionsaktiven oder Schwermetallionen im Ionenaustausch bindenden Stoff mit einer Partikelgröße bis zu 50 µm bzw. einer Agglomeratgröße bis zu 100 µm besteht.


**Claims**

**1.** Polyolefin-based electrical insulations, in particular for cables and leads, for medium and high voltage from approximately 10 kV having an additive to retard water trees, characterised in that the additive consists exclusively of a substance which is present in homogeneous distribution in a proportion of 0.1 to 4% by weight, as compared with the total weight, is hydrophilic, is active in adsorbing heavy metal ions or binds heavy metal ions by ion exchange and has a particle size of up to 50 µm or an agglomerate size of up to 100 µm.

**2.** Electrical insulations according to claim 1, characterised in that the additive is pyrogenic or precipitated silicic acid.

**3.** Electrical insulations according to claim 1, characterised in that the additive is aluminium oxide, aluminium hydrated oxide having a large active surface or aluminium silicate.

**4.** Electrical insulations according to one of the claims 1 to 3, characterised in that the additive is a synthetic product.

**5.** Electrical insulations according to one or several of the claims 1 to 4, characterised in that the proportion of the additive amounts to 0.5 to 2% by weight.

**6.** Electrical insulations according to one or several of the claims 1 to 5, characterised in that the particle size of the additive amounts to up to 20 µm.

**7.** Method for producing electrical insulations according to one or several of the claims 1 to 6, characterised in that the additive together with phlegmatizing means, preferably in the form of a paste, is incorporated into the polyolefins.

**8.** Cable or lead for medium and high voltage from approximately 10 kV having a polyolefin-based electrical insulation which contains an additive to retard water trees, characterised in that the additive consists exclusively of a substance which is present in homogeneous distribution in a proportion of 0.1 to 4% by weight, as compared with the total weight of the insulation, is hydrophilic, is active in adsorbing heavy metal ions or binds heavy metal ions by ion exchange and has a particle size of up to 50 µm or an agglomerate size of up to 100 µm.

**Revendications**

1. Isolants électriques à base de polyoléfines, utilisés notamment dans des câbles et des lignes, pour une moyenne tension et une haute tension à partir d'environ 10 kV, et comportant un additif servant à retarder la formation d'arborescences d'eau, caractérisés par le fait que l'additif est constitué exclusivement, réparti de façon homogène, de 0,1 à 4 % en poids, rapportés au poids total, d'une substance hydrophile active par adsorption sur des ions de métaux lourds ou fixant des ions de métaux lourds par échange d'ions, et ayant une dimension de particule allant jusqu'à 50 μm ou une dimension des agglomérats jusqu'à 100 μm.

2. Isolants électriques suivant la revendication 1, caractérisés par le fait que l'additif est de l'acide silicique pyrogène ou précipité.

3. Isolants électriques suivant la revendication 1, caractérisés par le fait que l'additif est un oxyde ou un oxy-hydrate d'aluminium ayant une grande surface active ou du silicate d'aluminium.

4. Isolants électriques suivant l'une des revendications 1 à 3, caractérisés par le fait que l'additif est un produit synthétique.

5. Isolants électriques suivant une ou plusieurs des revendications 1 à 4, caractérisés par le fait que la proportion de l'additif est comprise entre 0,5 et 2 % en poids.

6. Isolants électriques suivant une ou plusieurs des revendications 1 à 5, caractérisés par le fait que la dimension des particules de l'additif va jusqu'à 20 μm.

7. Procédé pour fabriquer des isolants électriques suivant l'une ou plusieurs des revendications 1 à 6, caractérisé par le fait qu'on introduit l'additif conjointement avec des agents de stabilisation, de préférence sous la forme d'une pâte, dans les polyoléfines.

8. Câble ou ligne pour une moyenne tension et une haute tension à partir d'environ 10 kV, comportant un isolant électrique à base de polyoléfine, contenant un additif servant à retarder la formation d'arborescences d'eau, caractérisé par le fait que l'additif est constitué exclusivement, réparti de façon homogène, de 0,1 à 4 % en poids, rapportés au poids total de l'isolant, d'une substance hydrophile active par adsorption sur des ions de métaux lourds ou fixant des ions de métaux lourds par échange d'ions, et ayant une dimension de particule allant jusqu'à 50 μm ou une dimension des agglomérats allant jusqu'à 100 μm.